# EUROPÄISCHE PATENTANMELDUNG

(11) **EP 2 465 553 A1**
(43) Veröffentlichungstag der Anmeldung: **20.06.2012**
(21) Anmeldenummer: 10015621.5
(22) Anmeldetag: 14.12.2010
(51) Int. Cl.: A61M 1/30

(54) **Extrakorporale Blutbehandlungsvorrichtung**

(71) Anmelder: B. Braun Avitum AG, 34212 Melsungen (DE)
(72) Erfinder: Bock, Gerhard, 36289 Friedewald (DE); Meibaum, Jörn, 34225 Baunatal (DE)
(74) Vertreter: Arth, Hans-Lothar

(57) **Zusammenfassung**

Die Erfindung betrifft eine extrakorporale Blutbehandlungsvorrichtung mit
- einer Steuereinrichtung (50) zur Steuerung des Öffnens und Schließens einer Arterienleitung (2) bei im Wesentlichen gleichzeitigem Schließen und Öffnen einer Venenleitung (3) in einer Single-Lumen-Anordnung (10), mit welcher unbehandeltes Blut durch die Single-Lumen-Anordnung (10) und die Arterienleitung (2) abgezogen, durch eine Blutbehandlungseinrichtung (5) geleitet und behandeltes Blut durch die Venenleitung (3) und die Single-Lumen-Anordung(10) zurückgeführt wird,
und ist dadurch gekennzeichnet, dass
eine Steuerung (50) vorgesehen ist, mit welcher ein Phasenvolumen in Abhängigkeit der aktuellen Förderrate Q_{BP} der wenigstens einen Pumpe (40) während einer Phase selbsttätig auf einen vordefinierten Wert Vₛₒₗₗ einstellbar ist.

## Beschreibung

Die Erfindung betrifft eine extrakorporale Blutbehandlungsvorrichtung gemäß dem Oberbegriff des Patentanspruchs 1.

Eine solche Vorrichtung ist beispielsweise aus der DE 34 22 375 A1 bekannt. Die dortige Vorrichtung weist einen Single-Lumen Katheter, durch welchen die extrakorporale Blutbehandlungsvorrichtung an den Blutkreislauf eines zu therapierenden Patienten anschließbar ist. Durch den einlumigen Anschluss des Patienten an die Vorrichtung ist es notwendig, dass darüber sowohl die Blutentnahme als auch das Reinfundieren des Blutes über diesen einlumigen Anschluss erfolgt.

Bei der DE 34 22 375 A1 sind mehrere Möglichkeiten der extrakorporalen Anordnung von Schläuchen, Drucksensoren, Klemmen und Pumpen zu sehen. Entscheidend für all diese Anordnungen ist, dass sie zum einen ein Arterienleitungsbehälter nach einer arteriellen Schlauchklemme und zum anderen ein Venenleitungsbehälter vor einer venösen Schlauchklemme aufweisen. Beide Behälter bilden einen Blutspeicher, so dass auch bei Verschluss einer der beiden Schlauchklemmen kontinuierlich Blut über den Dialysator gefördert werden kann. Die Steuerung zwischen arterieller Phase, in welcher dem Patienten das Blut entnommen und der Blutbehandlungsvorrichtung zugeführt wird, und venöser Phase, in welcher dem Patienten das entnommene Blut reinfundiert wird, geschieht in Abhängigkeit eines arteriellen und eines venösen Drucksensors, welche an dem Arterienleitungsbehälter bzw. Venenleitungsbehälter angebracht sind.

Dabei ist während der arteriellen Phase die arterielle Schlauchklemme geöffnet und die venöse geschlossen. Eine Blutpumpe fördert dabei das zu reinigende Blut durch das Schlauchsystem in den Dialysator und weiter in den Venenleitungsbehälter gegen die geschlossene venöse Schlauchklemme. Sobald in dem Venenleitungsbehälter der Druck eine vorgegebenen oberen Grenzwert erreicht hat, wird die arterielle Phase beendet und die venöse Phase begonnen, indem die venöse Schlauchklemme geöffnet und die arterielle geschlossen wird. Durch die Blutpumpe wird nun Blut aus dem Arterienleitungsbehälter in den Dialysator und weiter in den Venenleitungsbehälter gepumpt, während aus dem Venenleitungsbehälter gereinigtes Blut in den Patienten reinfundiert wird. Dies geschieht solange bis der sich in dem Arterienleitungsbehälter aufbauende Unterdruck einen unteren Grenzwert erreicht hat. Nun wird wiederum die arterielle Schlauchklemme geöffnet und die venöse geschlossen, so dass nunmehr die venöse Phase beendet ist und eine neue Phase, bestehend aus erneuter arterieller und erneuter venöser Phase, beginnt.

Grundsätzlich arbeitet die in der DE 34 22 375 A1 beschriebene Vorrichtung bzw. das dort beschriebene Verfahren zufriedenstellen, solange keine Störungen im extrakorporalem Kreislauf , insbesondere im Schlauchsystem, auftreten. Eine solche Störung kann einerseits beispielweise ein Knick in der arteriellen bzw. venösen Schlauchleitung darstellen. Andererseits kann eine solche Störung auch in einem falschen Einstellen der Blutpumpengeschwindigkeit durch das Bedienpersonal seine Ursache haben, wobei unter einer solchen Störung auch der Start der Therapie angesehen werden soll. Solche Störungen können im Extremfall dazu führen, dass kein effektiver Blutfluss — aus dem Patientenkörper heraus und wieder hinein - mehr stattfindet. Im folgenden sei eine solche Störung beispielhaft kurz anhand eines Knickes in der venösen Schlauchleitung beschrieben:

Durch einen Knick in der venösen Schlauchleitung während der venösen Phase erhöht sich dort der Flusswiderstand, wodurch der Abfluss des gereinigten Blutes in den Patienten abnimmt und sich der Venenleitungsbehälter entsprechend langsamer entleert. Dies hat zur Folge, dass sich das arterielle Phasenvolumen über mehrere Phasen verkleinert, weil bei gleichem Blutfluss über den Dialysator bis zum Erreichen des untere Druckgrenzwertes in dem Arterienleitungsbehälter sich der Venenleitungsbehälter nicht entsprechend entleert hat. Demzufolge wird der obere Druckgrenzwert in dem Venenleitungsbehälter während der folgenden Phasen immer früher erreicht. Das Gesamtsystem beginnt demzufolge zwischen arterieller und venöser Phase zu oszillieren, ohne dass ein effektiver Blutfluss durch die extrakorporale Blutreinigungsmachine stattfindet. Daher erfolgt in solchen Situationen, insbesondere auch unter Berücksichtigung des weiter unten beschriebenen Totvolumens, kein Reinfundieren bereits gereinigten Blutes in den Patienten. Somit findet in diesen Situationen mit in der DE 34 22 375 A beschriebenen extrakorporalen Blutreinigungsmachine effektiv keine Therapie mehr statt. Dies kann nur unterbunden werden, wenn das Bedienpersonal regulierend eingreift.

Es ist daher Aufgabe der Erfindung, eine Vorrichtung nach dem Oberbegriff des Patentanspruchs 1 zur Verfügung zu stellen, mit welcher trotz auftretender Störungen im Blutflusssystem die Therapie ohne korrigierendes Eingreifen des Bedienpersonals selbsttätig mit effektivem Blutfluss fortgesetzt wird.

Gelöst wird diese Aufgabe durch eine Vorrichtung mit den Merkmalen des Patentanaspruchs 1. Vorteilhafte Ausgestaltungen der Erfindung finden sich in den Unteransprüchen.

Bei den im folgenden verwendeten Begriffen arteriellen und venösen Phase wird die gleiche Definition zugrundegelegt, wie sie eingangs für die DE 34 22 375 A1 beschrieben sind, wobei hier anstatt arterieller und venöser Schlauchklemmen allgemeiner von Arterienleitungs- bzw. Venenleitungs-Schließeinrichtungen ausgegangen wird.

Für den effektiven Betrieb von Blutbehandlungsvorrichtungen ist von Bedeutung, dass das behandelte akkumulierte Blutvolumen so groß wie möglich ist. Durch die Besonderheit beim Einlumen-oder auch Single-Lumen-Betrieb reduziert ein Volumen, welches durch den einlumigen Anschluss der Blutbehandlungsvorrichtungen an das Gefäßsystem des Patienten definiert ist und als Totvolumen bezeichnet wird, das behandelte Blutvolumen. Da das bereits gereinigte Blut in dem einlumigen Zugang zum Patienten, also dem Totvolumen, in der arteriellen Phase wieder extrakorporal dem Dialysator zugeführt wird, sinkt die Effektivität der Behandlung. Das in diesem Totvolumen V_{Tot} enthaltene Blut wird Rezirkulationvolumen genannt, weil während jeder arteriellen Phase eine gewisse Blutmenge aufgrund des bestehenden Totraums im extrakorporalen Kreislauf nicht in den Patentientenkörper reinfundiert wird. Dieses Totvolumen ist durch den einlumigen Patientenzugang an den sich ein Y-Stück zum Zusammenführen des arteriellen und venösen Schlauchsystems anschließt, bedingt. Grundsätzlich gilt, je höher die Rezirkulation, desto geringer ist die Clearance-Leistung. Als Normbereich gilt eine Rezirkulationsanteil kleiner 10% des Phasenvolumens. Eine weitere Größe beim Einnadel-/Einlumen-Betrieb ist das Phasenvolumen V_{Phase}. Das Phasenvolumen V_{Phase} ergibt sich aus dem Blutvolumen in der arteriellen Phase V_{Art_Phase}, das dem Patienten entnommen wird, oder dem Blutvolumen in der venösen Phase V_{Ven_Phase}, das dem Patienten wieder zurückgegeben wird. Das Blutvolumen der arteriellen und venösen Phase unterscheidet sich durch die Ultrafiltration, die im Dialysator dem Blut entzogen wird.

Das effektive dem Dialysator neu aus dem Patientenkörper während einer Phase zugeführte Blutvolumen V_{eff} Phase berechnet daher sich zu:
V_{eff.} = V_{Art_Phase} - V_{Tot.}

Die Ermittlung des Phasenvolumens V_{Phase} erfolgt durch Multiplikation der Förderate Q_{BP} der Blutpumpe und der Zeitspanne t_{Phase} einer Phase zu:
V_{Phase} = Q_{BP} * t_{Phase}
wobei sich die Zeitspanne t_{Phase} einer Phase aus der Addition der Zeitspanne der arteriellen Phase t_{Art_Phase} und der Zeitspanne der venösen Phase t_{Ven_Phase} ergibt zu:
t_{Phase} = t_{Art_Phase} + t_{Ven_Phase}

Zu unterscheiden von der Förderrate Q_{BP} der Blutpumpe sind der arterielle Fluss Q_{Art_Blut} sowie der venöse Fluss Q_{Ven}__{Blut}, die ebenfalls entsprechend nachfolgenden Gleichungen mit dem Phasenvolumens **V_{Phase}** zusammenwirken. Es gilt:
V_{Phase} = V_{Art_Phase} ;V_{Ven_Phase}, wobei mit
V_{Art_Phase} = Q_{Art_Blut} * t_{Art_Phase} und
V_{Ven_Phase} = Q_{Ven_Blut} * t_{Ven_Phase}

für V_{Phase} folgender Zusammenhang gilt:
V_{Phase} = QBP * (t_{Art_Phase} + t_{Ven_Phase})

Das akkumulierte Blutvolumen das dem Patienten während der Therapie entnommen und wieder reinfundiert wird ergibt sich zu:
V_{Therapie} = Q_{Art_Blut} * t_{Art_Phase} * t_{Therapie}/ (t_{Art_Phase} + t_{Ven_Phase})

während das gesamte Totvolumen V_{Tot_Gesamt} sich über die gesamte Therapie zu
V_{Tot_Gesamt} = V_{Tot} * t_{Therapie}/ (t_{Art_Phase} + t_{Ven_Phase})

### aufsummiert.

Für die effektive behandelte Blutmenge beim Einnadel-/Einlumen-Betieb hat es sich daher erfindungagemäß als vorteilhaft erwiesen, das Phasenvolumen auf Grundlage von dem verwendeten Gefäßzugang vorzugeben und den Blutfluss kontinuierlich auf einen vordefinierten bzw. vorgegebenen Wert einzustellen. Damit ist sichergestellt, dass bei vorgegeben Gefäßzugang der optimale Blutfluss erreicht wird, bei dem der Rezirkulationsanteil beispielsweise kleiner 10% ist. Damit verbessert diese Erfindung die Qualität der Therapie und trägt zur höchst möglichem behandelten Blutmenge bei. Realisiert wird dies dadurch, dass in der erfindungsgemäßen Vorrichtung eine Steuereinrichtung vorgesehen ist, mit welcher ein Phasenvolumen in Abhängigkeit der aktuellen Förderrate Q_{BP} der wenigstens einen Blutpumpe während einer Phase selbsttätig auf einen vordefinierten bzw. vorgegebenen Wert Vₛₒₗₗ einstellbar ist.

Dazu wird das während einer Phase geförderte Blutvolumen Vᵢₛₜ bestimmt und mit dem vordefinierten bzw. vorgegebenen Wert Vₛₒₗₗ verglichen. Sofern keine Differenz zwischen Vᵢₛₜ Vₛₒₗₗ festgestellt werden kann, läuft die Therapie wie in der Phase, für die zuletzt Vᵢₛₜ bestimmt wurde, weiter. Wird allerdings eine Differenz ΔV = Vᵢₛₜ - Vₛₒₗₗ # 0 festgestellt wird durch die Steuereinrichtung der erfindungsgemäße Vorrichtung veranlasst, dass die Förderrate der Blutpumpe entsprechend geändert wird, so dass in der nachfolgenden Phase das Phasenvolumen in Richtung des vordefinierten bzw. vorgegebenen Wert Vₛₒₗₗ beeinflusst wird.

Dabei ist zu berücksichtigen, dass sowohl die Arterienleitungs-Schließeinrichtung als auch die Venenleitungs-Schließeinrichtung immer noch bei Erreichen der durch die den Arterien- bzw. Venenleitungsbehälter zugeordneten Sensoreinrichtungen erfassten Grenzwerte geöffnet bzw. geschlossen werden. Liegt allerdings das während einer Phase geförderte Blutvolumen Vᵢₛₜ unter dem Sollwert Vₛₒₗₗ, wird entgegen der zu erwartenden Steigerung eine Senkung der Förderrate der Pumpe durchgeführt. Dies hat seine Ursache darin, dass das Nichterreichen des Sollwertes Vₛₒₗₗ nicht in einer zu geringen Förderrate der Pumpe liegt, sondern daran, dass der vorgegebenen Grenzwert im Arterien- bzw. Venenleitungsbehälter zu früh erreicht wurde und deshalb die arterielle bzw. venöse Phase beendet, obwohl das zu fördernde Blutvolumen Vₛₒₗₗ in dieser Phase noch nicht erreicht wurde. Deshalb wird erfindungsgemäß durch die Steuereinrichtung das Phasenvolumen in Abhängigkeit der aktuellen Förderrate Q_{BP} der wenigstens einen Pumpe dahingehend auf einen vordefinierten Wert Vₛₒₗₗ eingestellt, dass durch eine Erniedrigung der Förderrate Q_{BP} die Zeitspanne vergrößert wird, in der im Arterien- bzw. Venenleitungsbehälter der vorgegebene Grenzwert erreicht wird. In dieser nun vergrößerten Zeitspanne der arteriellen bzw. venösen Phase hat das System nun mehr Zeit den entsprechenden vordefinierten Wert Vₛₒₗₗ für das Phasenvolumen zu erreichen. Liegt in den darauffolgenden Phasen das Phasenvolumen immer noch unterhalb dieses vordefinierten Wertes Vₛₒₗₗ wird die Förderrate der Pumpe nochmals entsprechend gesenkt. Um eine entsprechend gleichmäßige Therapie zu erzielen, ist es natürlich auch möglich, die Förderate Q_{BP} der wenigstens einen Pumpe entsprechend zu erhöhen, wenn das während einer Phase geförderte Blutvolumen Vᵢₛₜ über dem Sollwert Vₛₒₗₗ liegt.

Neben den bereits eingangs genannten Einflussgrößen wie Störungen im Schlauchsystem und in der Förderrate der Pumpe hat auch eine Viskositätsänderung des Blutes Auswirkungen auf das Phasenvolumen. Auch solche Störungen sind mit der erfindungsgemäßen Vorrichtung in ebenso in einfacher Weise zu handhaben wie die Einstellung des Phasenvolumens mit Hilfe der erfindungsgemäßen Vorrichtung beim Starten einer Blutbehandlung bei einem Patienten.

In der Praxis hat es sich bewährt, die Steuereinrichtung als Regelkreis auszubilden, bei dem das Phasenvolumen selbsttätig auf einen Sollwert regelbar ist, wobei die Abweichung ΔV eines Istwertes Vᵢₛₜ des Phasenvolumens von dessen Sollwert Vₛₒₗₗ zur Regelung verwendet wird. Vorteilhafterweise ist der Regler des Regelkreises als P-Regler, I-Regler, D-Regler, PI-Regler, PD-Regler, PID-Regler, Zweipunktregler, Dreipunktregler, Mehrpunktregler bzw. Fuzzy-Regler ausgebildet ist. Durch die große Auswahl dieser verschiedenen Reglertypen ist es in einfacher Weise möglich, die Regelung auch sehr fein abzustimmen, so dass bereits kleinste ΔV-Werte zu einem Eingreifen der Steuerung führen und somit das Phasenvolumen über die gesamte Therapie annähernd konstant gehalten werden kann, wodurch eine optimale Therapie gewährleistet wird. Auf die einzelnen mathematischen Formulierungen der verschiedenen Regler wird an dieser Stelle verzichtet, da die Ausgestaltung eines entsprechenden Reglers und der dort zu verwendeten konstanten Faktoren je nach verwendeter erfindungsgemäßer Vorrichtung variieren kann und deren Bestimmung im Ermessen des Fachmanns liegt.

Weiterhin vorteilhaft ist es, den Sollwert Vₛₒₗₗ des Phasenvolumens mindestens fünfmal, vorzugsweise mindestens zehnmal so groß zu halten wie das Totvolumen der Single-Lumen-Anordnung. Dadurch ist sichergestellt, dass ein möglichst großes effektives Blutvolumen über die gesamte Therapie gereinigt wird.

In einer weiteren Ausführungsform der Erfindung hat es sich als sinnvoll erwiesen die Regelung des Phasenvolumen zeitverzögert durchzuführen. Dazu ist die Steuereinrichtung derart ausgebildet, dass sie die Regelung auf das Phasenvolumen unmittelbar nach Detektion einer Abweichung ΔV # 0 des Istwertes Vᵢₛₜ des Phasenvolumens von dessen Sollwert Vₛₒₗₗ um eine vorgegebenen Totzeit T_{Tot} verzögert. Durch diese Maßnahme ist vermieden, dass die Regelung unnötigerweise anspricht, wenn die Abweichung ΔV # 0 innerhalb eines Toleranzbereichs liegt. Dies kann beispielsweise durch systembedingte Einflüsse, wie beispielsweise bei Verwendung einer konstruktionsbedingt nicht gleichmäßig fördernden Rollenpumpe, oder aber durch nur kurzzeitige Störungen, wie beispielsweise eines nur kurzzeitigen Knickes in der Arterien- und/oder Venenleitung, bedingt sein. Die Totzeit T_{Tot} sollte deshalb nicht absolut festgelegt sein, sonder sich an einer bestimmten Anzahl vorgegebener Phasen, bestehend wie bereits zuvor beschrieben jeweils aus einer arteriellen und einer venösen Phase.

In der Praxis hat es sich deshalb als vorteilhaft erwiesen, diese Totzeit T_{Tot} wenigsten als zwei solcher Phasen, bevorzugt als vier solcher Phasen und maximal als zehn solcher Phasen zu definieren.

Um den apparativen Aufwand zu minimieren ist es vorteilhaft, nur eine Pumpe zu verwenden, die zwischen dem Arterienleitungsbehälter und dem Venenleitungsbehälter anzuordnen ist. Dadurch ist eine definierte Blutförderung sowohl in der arteriellen als auch in der venösen Phase mit nur einer Pumpe im System gewährleistet, was nicht nur im Hinblick auf Herstellungs- und Anschaffungskosten, sondern auch im Hinblick auf Wartungsarbeiten sehr ökonomisch ist.

In einer besonderen Ausgestaltung der Erfindung hat es sich als vorteilhaft erwiesen, dass die den Arterienleitungs- und Venenleitungsbehälter zugeordneten Sensoreinrichtungen als Drucksensoren ausgebildet sind. Damit kann in einfacher Weise über auftretende Unter- bzw. Überdrücke im Arterienleitungs- bzw. Venenleitungsbehälter deren Füllstand und mit Hilfe der Steuereinrichtung bzw. der Förderrate Pumpe und der zwischen Öffnen und Schließen vergangen Zeit bzw. des in dieser Zeit geförderten Blutes der Istwert Vᵢₛₜ für das Phasenvolumen bestimmt werden.

Weitere Ziele, Vorteile, Merkmale und Anwendungsmöglichkeiten der vorliegenden Erfindung ergeben sich aus der nachfolgenden Beschreibung der Ausführungsbeispiele anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen und deren Rückbeziehung.

Es zeigt:
- Figur 1:: eine schematische Darstellung eines Ausführungsbeispiels einer erfindungsgemäßen Vorrichtung mit bereits angeschlossenen Patienten,
- Figur 2:: eine schematische Darstellung eines Ausführungsbeispiels einer an ein Gefäßsystem eines Patienten angeschlossenen Single-Lumen-Anordnung,
- Figur 3:: Darstellung der Abhängigkeit des Phasenvolumens vom Fördervolumen der Blutpumpe für verschiedene Blutviskositäten,
- Figur 4:: Beispiel einer Signalschwankung des Phasenvolumens in Abhängigkeit von der Zeit bei Verwendung einer Rollenpumpe.

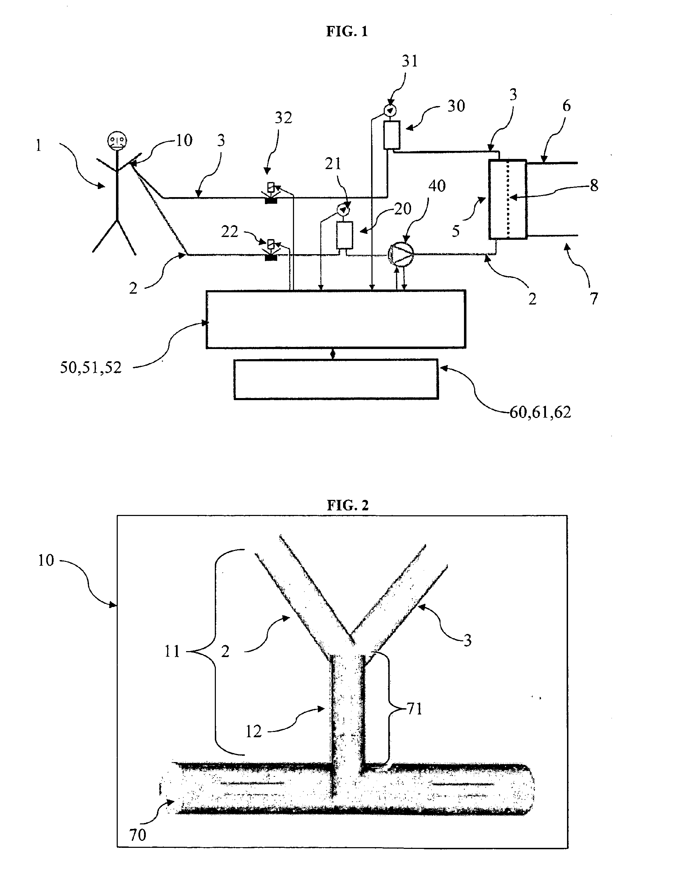

In Figur 1 ist ein Ausführungsbeispiel einer erfindungsgemäßen extrakorporale Blutbehandlungsvorrichtung schematisch dargestellt. In dieser Darstellung ist ein zu therapierender Patient 1 durch eine Single-Lumen-Anordnung 10 an die Vorrichtung angeschlossen. Die Single-Lumen-Anordnung 10 kann dabei den Zugang zum Patienten beispielsweise mittels einer Nadel oder einer flexiblen Verweilkanüle realisieren. Die Single-Lumen Anordnung 10, wie insbesondere aus Figur 2 ersichtlich, ist als ein Y-Stück 11 ausgebildet, bestehend aus dem Beginn einer Arterienleitung 2 und dem Ende einer Venenleitung 3 der extrakorporale Blutbehandlungsvorrichtung sowie einem Patientenzugang 12.

Während der Therapie wird dem Gefäßsystem 70 des Patienten 1 mittels des Patientenzugangs 12 der Single-Lumen-Anordnung 10 Blut entnommen. Aus dem Patientenzugang 12 gelangt das zu reinigende Blut wie aus Figur 1 ersichtlich über die Arterienleitung 2 in einen Arterienleitungsbehälter 20, wobei es eine in der Arterienleitung 2 angeordnete Arterienleitungs-Schließeinrichtung 22 passiert. Von dem Arterienleitungsbehälter 20 wird das Blut nun weiter entlang der sich bis zu einer Blutbehandlungseinrichtung 5 erstreckenden Arterienleitung 2 gefördert. In diesem Ausführungsbeispiel erfolgt die Förderung des Blutes aus dem Arterienleitungsbehälter 20 mittels eine Pumpe 40, die beispielsweise als Rollenpumpe ausgebildet sein kann. In der Blutbehandlungseinrichtung 5 findet die eigentlich Reinigung des Blutes statt, indem über Diffusion und/oder Konvektion dem Blut toxische Substanzen und/oder Flüssigkeit entzogen wird. Die Reinigung des Blutes erfolgt hierbei über eine in der Blutbehandlungseinrichtung 5 angeordneten semipermeable Membran 8. Das zu reinigende Blut fließt in diesem Ausführungsbeispiel im Gegenstrom zu einer über einen Dialysierflüssigkeitszulauf 6 der Blutbehandlungseinrichtung 5 zugeführten Dialysierflüssigkeit über die semipermeable Membran. Über einen an der Blutbehandlungseinrichtung 5 angeordneten Dialysierflüssigkeitsablauf 7 wird die mit den aus dem zu reinigeneden Blute entfernten Toxinen angereicherte verbrauchte Dialysierflüssigkeit einer nichtdargestellten Sammeleinrichtung zugeführt. Das gereinigte Blut wird der Blutbehandlungseinrichtung 5 mittels einer angeordneten Venenleitung 3 entnommen und in einen Venenleitungsbehälter 30 gefördert. Von dort wird das gereinigte Blut über eine Fortsetzung der Venenleitung 3, wobei es eine in der Venenleitung 3 angeordnetes Venenleitungs-Schließeinrichtung 32 passiert, wieder der Single-Lumen-Anordnung 10 zugeführt. Hier wird nun das gereinigte Blut über den Patientenzugang 12 des Y-Stücks 11 fast vollständig wieder in das Gefäßsystem 70 des zu therapierenden Patienten 1 reinfundiert.

Bei einer extrakorporalen Blutbehandlungsvorrichtung mit einer Single-Lumen-Anordnung 10 als Zugang zum Gefäßsystem 70 des Patienten 1 ist es allerdings aufgrund dieser speziellen Ausgestaltung nicht möglich, dem zu therapierenden Patienten 1 gleichzeitig sowohl zu reinigendes Blut zu entnehmen als auch gereinigtes Blut wieder in den Patienten 1 zu reinfundieren. Dazu sind extrakorporale Blutbehandlungsvorrichtung mit Single-Lumen-Anordnung 10 wie in diesem Ausführungsbeispiel beschrieben mit speziellen Steuer- und Sensorelementen ausgestattet, die es ermöglichen phasenweise abwechselnd dem Patienten 1 zu reinigendes Blut zu entnehmen und gereinigtes Blut zu reinfundieren. Zu diesen Steuer- und Sensorelementen gehören neben den bereits erwähnten Arterienleitungs- und Venenleitungs-Schließeinrichtungen 22, 32 und der Pumpe 40 dem Arterienleitungsbehälter 20 bzw. dem Venenleitungsbehälter 30 zugeordnete Sensoreinrichtungen 21, 31, durch die eine Aussage über den Füllstand in dem jeweiligen Behälter 20,30 gewonnen werden kann. Weiterhin gehören dazu noch eine Steuereinrichtung 50, eine Überwachungseinrichtung 51, eine Speichereinrichtung 52, eine Anzeigeeinrichtung 60, eine Eingabeeinrichtung 61 sowie eine Kommunikationseinrichtung 62.

Die Kommunikationsrichtung dieser einzelnen Steuer- und Sensorelementen ist in Figur 1 durch Pfeile dargestellt. So empfängt die aus Steuereinrichtung 50, Überwachungseinrichtung 51 und Speichereinrichtung 52 bestehende Gruppe Daten von den Sensorelementen 31 und 21. Aufgrund dieser Daten können die Elemente dieser Gruppe und insbesondere die Steuereinrichtung 50 sowohl die Schließeinrichtungen 22 und 32 als auch die Pumpe 40 Anweisungen übermitteln, ihren aktuellen Betriebszustand zu ändern. Ferner können die Steuereinrichtung 50, die Überwachungseinrichtung 51 und die Speichereinrichtung 52 mit der Anzeigeeinrichtung 60, der Eingabeeinrichtung 61 sowie der Kommunikationseinrichtung 62 bidirektional Daten und Informationen austauschen.

Nachfolgend wird nun die Funktionsweise des Ausführungsbeispiels gemäß Figur 1 beschrieben, wobei nicht auf den Therapiebeginn Bezug genommen wird, sondern auf die Arbeitsweise während einer schon laufenden Therapie. Beginnend mit einer arteriellen Phase stellt sich die Situation im normalen störungsfreien Betrieb wie folgt dar:
Während der arteriellen Phase ist die die Arterienleitungs-Schließeinrichtung 22 geöffnet, so dass das dem Patienten 1 mittels der Single-Lumen-Anordnung 4 entnommene Blut durch die Arterienleitung 2 in den Arterienleitungsbehälter 20 fließen kann. Gleichzeitig fördert die Pumpe 40 das bereits in dem Arterienleitungsbehälter 20 befindliche Blut weiter durch die Fortsetzung des Arterienleitung 2 in die als Dialysator ausgebildete Blutbehandlungseinrichtung 5, wo dem Blut toxischen Substanzen mittels Diffusion und/oder

Konvektion entzogen sowie das Gewicht des Patienten durch Ultrafiltration verringert wird. Von dort gelangt das nunmehr bereits behandelte Blut über die Venenleitung 3 in den Venenleitungsbehälter 30. Da in der arteriellen Phase die Venenleitungs-Schließeinrichtung 32 geschlossen ist, kann dass im Venenleitungsbehälter 30 befindlich Blut nicht abfließen und über die Single-Lumen-Anordnung in das Gefäßsystem 70 des Patienten 1 reinfundiert werden. Aufgrund der geschlossenen Venenleitungs-Schließeinrichtung 32 füllt sich daher der Venenleitungsbehälter 30 mit bereits behandeltem Blut, bis ein bestimmte durch einen Grenzwert definierter oberer Füllstand im Venenleitungsbehälter 30 erreicht ist. Dieser Grenzwert wird im vorliegenden Ausführungsbeispiel gemäß Figur 1 durch die als Drucksensor ausgebildete Sensoreinrichtung 31 anhand eines vordefinierten Überdruckes innerhalb des Venenleitungsbehälters 30 erfasst. Wird dieser Überdruckwert durch den Drucksensor 32 an die Steuereinrichtung 50 übermittelt, veranlasst diese im wesentlichen gleichzeitig ein Schließen der Arterienleitung 2 durch die Arterienleitungs-Schließeinrichtung 22 und ein Öffnen der Venenleitung 3 durch die Venenleitungs-Schließeinrichtung 32.

Damit ist die arterielle Phase beendet und es beginnt die venöse Phase, in welcher dem Patienten 1 bereits behandeltes Blut reinfundiert wird. In der venösen Phase ist die Venenleitung 3 nicht mehr durch die Venenleitungs-Schließeinrichtung 32 geschlossen, so dass das in dem Venenleitungsbehälter 30 befindlich bereits behandelte Blut in das Gefäßsystem 70 des Patienten reinfundiert wird. Der Venenleitungsbehälter 30 entleert sich dabei aufgrund der andauernden Förderung des Blutes durch die Pumpe 40. Durch deren Förderung wird nicht nur der Venenleitungsbehälter 30 kontinuierlich weiter mit Blut beliefert, sondern auch der Arterienleitungsbehälter 20 entleert. Dies geschieht solange bis ein bestimmter durch einen Grenzwert definierter unterer Füllstand im Arterienleitungsbehälter 20 erreicht ist. Dieser Grenzwert wird im vorliegenden Ausführungsbeispiel gemäß Figur 1 durch die als Drucksensor ausgebildete Sensoreinrichtung 21 anhand eines vordefinierten Unterdruckes innerhalb des Arterienleitungsbehälters 20 erfasst. Wird dieser Unterdruckwert durch den Drucksensor 21 an die Steuereinrichtung 50 übermittelt veranlasst diese im wesentlichen gleichzeitig ein Schließen der Venenleitung 3 durch die Venenleitungs-Schließeinrichtung 32 und ein Öffnen der Arterienleitung durch die Arterienleitungs-Schließeinrichtung 22.

Damit ist nun auch die venöse Phase beendet und während dieser aus arterieller und venöser Phase bestehenden Phase hat die Pumpe 40 im wesentlichen innerhalb bestimmter

Toleranzgrenzen das Phasenvolumen Vᵢₛₜ= Vₛₒₗₗ gefördert. Diese bis jetzt beschrieben Funktionsweise stellt die gewünschte Funktionsweise ohne Störung dar. Bei Beginn der nächsten Phase bestehend aus arterieller und venöser Phase befindet sich im Patientenzugang 12 des Y-Stücks 11 der Single-Lumen-Anordnung 4 bereits behandeltes Blut, welches nun in der arteriellen Phase wieder dem Reinigungskreislauf in der extrakorporalen Blutreinigungsvorrichtung zugeführt wird, ohne dass es zuvor dem Patienten 1 wieder reinfundiert wurde. Man bezeichnet das Volumen des Patientenzugangs 12 daher auch als Totvolumen 71, da bei einer extrakorporalen Blutreinigungsvorrichtung mit einer Single-Lumen-Anordnung 4 es unvermeidbar ist, dass das bei Beendigung der venösen Phase sich in diesem Totvolumen 71 befindliche Blut, ohne zuvor in das Gefäßsystem 70 des Patienten 1 reinfundiert worden zu, sein nochmals einem Reinigungszyklus unterworfen wird.

Es ist daher von besonderem Interesse, das Verhältnis von Phasenvolumen Vᵢₛₜ zu dem Totvolumen (71) V_{Tot} möglichst groß bzw. möglichst nah an dem Wert Vₛₒₗₗ/V_{Tot} zu halten. Deshalb ist es wünschenswert, dass bei auftretenden Störungen, besonders im Schlauchsystem der extrakorporalen Blutreinigungsvorrichtung die Vorrichtung möglichst schnell selbsttätig entsprechende Aktionen ausführt. Nachfolgend soll die selbsttätige Regulierung dieses Ausführungsbeispiels anhand einer auf einem Knick in der Venenleitung 3 zwischen der Single-Lumen-Anordnung 4 und dem Venenleitungsbehälter 30 basierenden Störung im Schlauchsystem der extrakorporalen Blutreinigungsvorrichtung beginnend während einer venösen Phase beschrieben werden.

Durch den Knick an dieser Stelle in der Venenleitung 3 erhöht sich dort der Flusswiderstand für das Blut, dass aus dem Venenleitungsbehälter 30 entleert wird und strömt somit während der venösen Phase langsamer als ohne Knick. Gleichzeitig wir aber der Arterienleitungsbehälter 20 bei geschlossener Arterienleitungs-Schließeinrichtung 22 durch die andauernde Förderung der Pumpe 40 weiter entleert bis der vordefinierte Grenzwert für den Unterdruck in dem Arterienleitungsbehälter 20 erreicht ist. Nun wird durch die Steuereinrichtung 50 die Arterienleitungs-Schließeinrichtung 22 geöffnet und die Venenleitungs-Schließeinrichtung 32 geschlossen, so dass die venöse Phase beendet ist und die arterielle Phase beginnt. Dadurch dass während der venöse Phase durch den Knick eine langsamere Entleerung des Venenleitungsbehälters 30 als im ungestörten System stattgefunden hat, ist der Füllstand dort zu Beginn der arteriellen Phase im Vergleich zum ungestörten extrakorporalen Kreislauf höher, so dass bei unveränderter Förderate der Pumpe 40 der vordefinierte Grenzwert für den Überdruck in dem Venenleitungsbehälter 30 schneller erreicht wird. Dies bedeutete, dass das arterielle Phasenvolumen im Vergleich zu ungestörten System gering ist, da die Steuereinrichtung 50 die arterielle Phase beendet, wenn der Sensoreinrichtung 31 den vordefinierten Grenzwert für den Überdruck in den Venenleitungsbehälter 30 erfasst und an die Steuereinrichtung 50 weiterleitet. In der darauf folgenden venösen Phase wiederholt sich dieses Szenario, bei anhaltender Störung. Dadurch dass die Entleerung des Venenleitungsbehälters 30 weiterhin verlangsamt erfolgt, ist der Füllstand im Venenleitungsbehälters 30 am Ende dieser venösen Phase noch höher als in der Phase zuvor, so dass in der nun folgende arteriellen Phase noch weniger Blut durch die Pumpe 40 in den Venenleitungsbehälter 30 gefördert würde. Ohne eine Eingreifen der erfindungsgemäßen Regelung würde dass System nun eskalieren und im Extremfall wären sowohl im Venenleitungsbehälter 30 als auch im Arterienleitungsbehälter 20 die vordefinierten Grenzwerte sofort nach Umschalten durch die Steuereinrichtung 50 von der arteriellen in die venöse Phase oder umgekehrt erreicht und es würde kein Phasenvolumen mehr gefördert. Um dem entgegenzuwirken wird mit Hilfe der Steuer-, Über- und Speichereinrichtungen 50, 51 und 52 die Abweichung ΔV des Istwertes Vᵢₛₜ des durch die Pumpe 40 während einer aus arterieller und venöser Phase gebildeten Phase geförderten Phasenvolumens von dem in der Speichereinrichtung 52 abgelegten Sollwert Vₛₒₗₗ bestimmt. Im vorliegenden Fall ist die Abweichung ΔV positiv und sofern sie außerhalb eines vordefinierten Toleranzbereiches liegt, greift nun der Regelkreis in das System ein und veranlasst die Steuereinrichtung 50 dazu, die Förderrate der Pumpe 40 um einen bestimmten Betrag zu senken. Durch die nunmehr geringere Förderrate der Pumpe 40 wird während einer venöse Phase nunmehr weniger Blutvolumen in den Venenleitungsbehälter 30 gefördert, so dass- ggf. erst nach mehrerer aus arterieller und venöser Phase bestehenden Zyklen bzw. Phasen - das durch die Entleerung des Venenleitungsbehälter 30 während einer venösen Phase definierte Blutvolumen größer ist als das in dieser venösen Phase durch die Pumpe 40 in den Venenleitungsbehälter 30 geförderte Blutvolumen. Durch diese Maßnahme erhöhen sich die absoluten Zeiten für die venöse und arterielle Phase, so dass obwohl die Förderrate der Pumpe 40 gesenkt wird, das in einer Phase bestehend aus arterieller und venöser Phase durch die Pumpe 40 geförderte Blutvolumen und damit das Phasenvolumen Vᵢₛₜ erhöht wird. Befindet sich das Phasenvolumen Vᵢₛₜ wieder im Toleranzbereich von Vₛₒₗₗ wirkt der Regler nicht mehr. Das Phasenvolumen Vᵢₛₜ wurde somit durch den Regelkreis selbsttätig ohne Einschreiten des Anwenders in den Toleranzbereich des Sollwerts Vₛₒₗₗ eingestellt.

Wird die Störung - vorliegender Knick in der Venenleitung 2 - entfernt greift auch dann der Regelkreis wieder ein. Der nunmehr geringerer Flusswiderstand hätte eine Erhöhung des Phasenvolumens zur Folge, so dass die Steuereinrichtung 50 durch den Regelkreis die Förderrate der Pumpe 40 erhöhen würde ,bis das Phasenvolumen Vᵢₛₜ wieder im Toleranzbereich des Sollwertes Vₛₒₗₗ liegt.

Somit ist es möglich, die Therapie mit der erfindungsgemäßen Vorrichtung aufrecht zu erhalten, solange im extrakorporalen Blutkreislauf noch eine Zirkulation stattfindet und die Störung das Schlauchsystem nicht komplett blockiert.

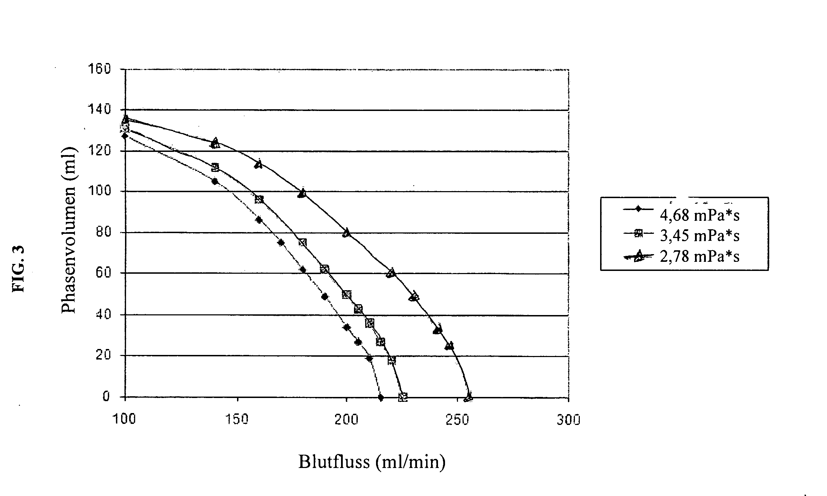

Als eine Störung im weiteren Sinn kann auch eine Änderung der Viskosität des Blutes des Patienten 1 während einer Therapie angesehen werden. Wie in Figur 3 dargestellt verhält sich nämlich für verschiedene Viskositäten das Phasenvolumen zum Blutfluss unterschiedlich. Viskositätsänderungen während einer Therapie treten natürlich auch durch den nicht zu vermeidenden Flüssigkeitsverlust des Patienten 1 auf. Auch solche Viskostätsänderungen verursachen eine Änderung des Flusswiderstandes im Schlauchsystem der Vorrichtung, die als Störung aufgefasst werden, da dadurch der Betrag des Verhältnisse von Vᵢₛₜ/V_{Tot} verkleinert wird und somit die Effektivität der Therapie verschlechtert wird. Auch eine solche durch Viskositätsänderung des Blutes während der Therapie des Patienten 1 kann auftretende Störung kann durch den Regelkreis entsprechend beeinflusst werde, so dass optimale Effektivität der Therapie erreicht wird.

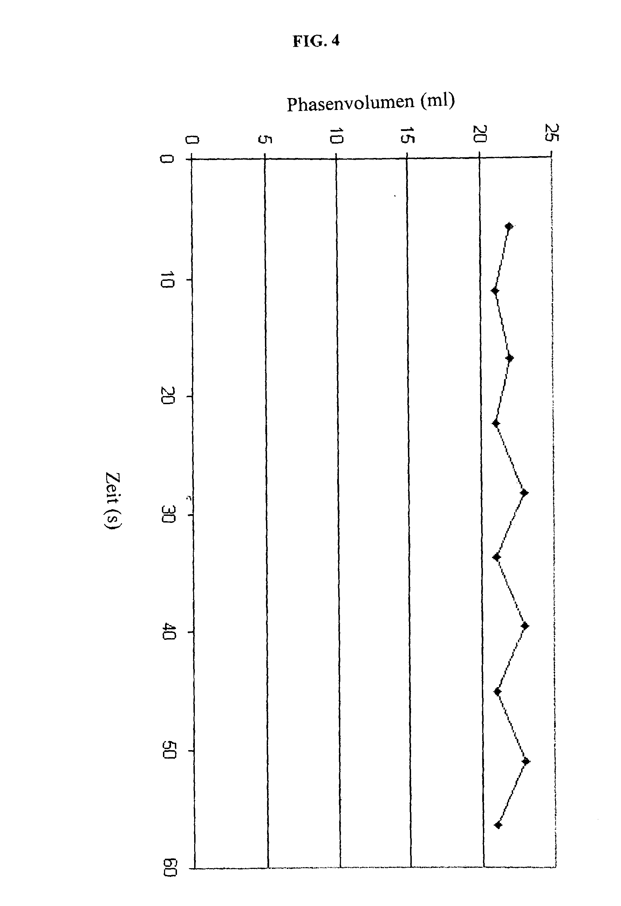

In Figur 4 ist beispielshaft das Verhalten des Phasenvolumens Vᵢₛₜ im ungestörten Zustand über die Zeit bei Verwendung einer im Bereich der extrakorporalen Blutbehandlung üblichen Rollenpumpe 40 dargestellt. Die dort gezeigten Schwankungen des Phasenvolumens Vᵢₛₜ, die durch die Konstruktion und die physikalischen Gegebenheiten einer Rollenpumpe 40 bedingt sind, sind der Grund dafür, dass nicht auf einen exakten Sollwert Vₛₒₗₗ geregelt wird, sondern auf einen Toleranzbereich oder Tunnel um einen solchen Sollwert. Der Hintergrund hierfür ist, dass durch die Verwendung einer Rollenpumpe Abweichungen des Phasenvolumens Vᵢₛₜ unvermeidlich sind. Solche Abweichung sollen aber nicht bereits dazu führen, dass das Phasenvolumen Vᵢₛₜ auf den Sollwert Vₛₒₗₗ geregelt wird. Erst wenn der Istwert des Phasenvolumens Vᵢₛₜ den vorgegebenen Toleranzbereich bzw. Tunnel verlässt, soll der Regelkreis eingreifen, um die bestmögliche Effektivität der Therapie bei bestmöglicher Wirtschaftlichkeit zu gewährleisten.

### Bezugszeichenliste:

- 1 -: Patient
- 2 -: Arterienleitung
- 3 -: Venenleitung
- 5 -: Blutbehandlungseinrichtung
- 6 -: Dialysierflüssigkeitzulauf
- 7 -: Dialysierflüssigkeitablauf
- 8 -: Membran
- 10 -: Single-Lumen-Anordnung
- 11 -: Y-Stück
- 12 -: Patientenzugang
- 20 -: Arterienleitungsbehälter
- 21 -: Sensoreinrichtung
- 22 -: Arterienleitungs-Schließeinrichtung
- 30 -: Venenleitungsbehälter
- 31 -: Sensoreinrichtung
- 32 -: Venenleitungs-Schließeinrichtung
- 40 -: Pumpe
- 50 -: Steuereinrichtung
- 51 -: Überwachungseinrichtung
- 52 -: Speichereinrichtung
- 60 -: Anzeigeeinrichtung
- 61 -: Eingabeeinrichtung
- 62 -: Kommunikationseinrichtung
- 70 -: Gefäßsystem
- 71 -: Totvolumen

## Patentansprüche

1. Extrakorporale Blutbehandlungsvorrichtung mit
- einer Steuereinrichtung (50) zur Steuerung des Öffnens und Schließens einer Arterienleitung (2) bei im Wesentlichen gleichzeitigem Schließen und Öffnen einer Venenleitung (3) in einer Single-Lumen-Anordnung (10), mit welcher unbehandeltes Blut durch die Single-Lumen-Anordnung (10) und die Arterienleitung (2) abgezogen, durch eine Blutbehandlungseinrichtung (5) geleitet und behandeltes Blut durch die Venenleitung (3) und die Single-Lumen-Anordung(10) zurückgeführt wird,
- einem Arterienleitungsbehälter (20), der in der Arterienleitung (2) vor der Blutbehandlungseinrichtung (5) angeordnet ist,
- einem Venenleitungsbehälter (30), der in der Venenleitung (3) hinter der Blutbehandlungseinrichtung (5) angeordnet ist,
- einer Sensoreinrichtung (31) zum Erfassen einer über einem Grenzwert liegenden Menge behandelten Bluts in dem Venenleitungsbehälter (30),
- einer Sensoreinrichtung (21) zum Erfassen einer unter einem Grenzwert liegenden Menge unbehandelten Bluts in dem Arterienleitungsbehälter (20),
- einer Arterienleitungs-Schließeinrichtung (22) zum Verschließen der Arterienleitung (2) bei im Wesentlichen gleichzeitigem Öffnen der Venenleitung (3) ansprechend auf ein erstes Signal, das von der Sensoreinrichtung (31) an eine Arterienleitungs-Schließeinrichtung (22) übermittelt wird,
- einer Venenleitungs-Schließeinrichtung (32) zum Verschließen der Venenleitung (3) bei im Wesentlichen gleichzeitigem Öffnen der Arterienleitung (2) ansprechend auf ein zweites Signal, das von der Sensoreinrichtung (21) an eine Venenleitungs-Schließeinrichtung (32) übermittelt wird und
- wenigstens einer Pumpe (40),
**dadurch gekennzeichnet, dass**
eine Steuerung (50) vorgesehen ist, mit welcher ein Phasenvolumen in Abhängigkeit des aktuellen Förderrate Q_{BP} der wenigstens einen Pumpe (40) während einer Phase selbsttätig auf einen vordefinierten Wert Vₛₒₗₗ einstellbar ist.

2. Vorrichtung nach Anspruch 1, **dadurch gekennzeichnet, dass** die Steuereinrichtung (50) als Regelkreis ausgebildet ist, bei dem das Phasenvolumen auf einen Sollwert Vₛₒₗₗ regelbar ist, wobei die Abweichung ΔV eines Istwertes Vᵢₛₜ des Phasenvolumens von dessen Sollwert Vₛₒₗₗ zur Regelung verwendet wird.

3. Vorrichtung nach Anspruch 2, **dadurch gekennzeichnet, dass** der Regler des Regelkreises als P-Regler, I-Regler, D-Regler, PI-Regler, PD-Regler, PID-Regler, Zweipunktregler, Dreipunktregler, Mehrpunktregler bzw. Fuzzy-Regler ausgebildet ist.

4. Vorrichtung nach einem der Ansprüche 2 oder 3, **dadurch gekennzeichnet, dass** der Sollwert Vₛₒₗₗ des Phasenvolumens mindestens fünfmal, vorzugsweise mindestens zehnmal so groß ist wie ein Totvolumen V_{Tot} (71) der Single-Lumen-Anordnung (10).

5. Vorrichtung nach einem der vorherigen Ansprüche, **dadurch gekennzeichnet, dass** die Steuereinrichtung (50) derart ausgebildet ist, dass sie die Regelung auf das Phasenvolumen unmittelbar nach Detektion einer Abweichung ΔV # 0 des Istwertes Vᵢₛₜ des Phasenvolumens von dessen Sollwert Vₛₒₗₗ um eine vorgegebenen Totzeit T_{Tot} verzögert.

6. Vorrichtung nach Anspruch 5, **dadurch gekennzeichnet, dass** die vorgegebenen Totzeit T_{Tot} wenigsten zwei Phasen, bevorzugt wenigsten vier Phasen und maximal zehn Phasen beträgt.

7. Vorrichtung nach einem der vorherigen Ansprüche, **dadurch gekennzeichnet, dass** die wenigstens eine Pumpe (40) zwischen dem Arterienleitungsbehälter (20) und dem Venenleitungsbehälter (30) angeordnet ist.

8. Vorrichtung nach einem der vorherigen Ansprüche, **dadurch gekennzeichnet, dass** die Sensoreinrichtungen (21, 31) als Drucksensoren ausgebildet sind.

9. Vorrichtung nach einem der vorherigen Ansprüche, **dadurch gekennzeichnet, dass** zur Bestimmung des Istwertes Vᵢₛₜ des Phasenvolumens die Förderrate der Q_{BP} der Pumpe 40 sowie die zwischen dem ein erstes Signal, das von der Sensoreinrichtung (31) an eine Arterienleitungs-Schließeinrichtung (22) übermittelt wird, und dem zweites Signal, das von der Sensoreinrichtung (21) an eine Venenleitungs-Schließeinrichtung (32) übermittelt wird, verwendet wird.
